# EUROPEAN PATENT APPLICATION

(11) **EP 2 233 080 A1**
(43) Date of publication of application: **29.09.2010**
(21) Application number: 10250604.5
(22) Date of filing: 26.03.2010
(51) Int. Cl.: A61B 17/04, A61B 17/072

(54) **Soft tissue graft preparation devices and methods**

(30) Priority: 27.03.2009 US 164036 P; 03.02.2010 US 699283
(71) Applicant: Tyco Healthcare Group LP, North Haven, CT 06473 (US)
(72) Inventor: May, Thomas C., MA 02093 (US); Hotter, Joseph, CT 06457 (US)
(74) Representative: Soames, Candida Jane

(57) **Abstract**

A soft tissue graft preparation device includes a handle portion, a body portion extending distally from the handle portion, and an end effector operatively connected to a distal end of the body portion. The end effector includes first and second members. The soft tissue graft preparation device also includes a filamentous material attachment structure that includes at least one filamentous material attached thereto, wherein the filamentous material attachment structure is placed in longitudinal juxtaposition to a tissue contacting surface of the first member and/or a tissue contacting surface of the second member.

## Description

### CROSS-REFERENCE TO RELATED APPLICATION

This patent application claims priority to, and the benefit of, U.S. Provisional Application Serial No. 61/164,036 filed on March 27, 2009 entitled "Soft Tissue Graft Preparation Devices and Methods" the disclosure of which is herein incorporated by reference in its entirety.

### BACKGROUND

### 1. Technical Field

The present disclosure relates to surgical devices and, more particularly, to surgical devices and methods for use in connection with soft tissue graft preparation.

### 2. Discussion of Related Art

A variety of surgical treatments involve the reconstruction of ligaments using grafts, including arthroscopic shoulder surgery, knee ligament reconstruction techniques and other orthopedic surgeries. Various grafting materials have been used in ligament reconstructions, including autologous grafts, also known as autografts (tissue transferred from one site to another in the same individual), allografts (donor tissue), xenografts (tissue from a species other than man) and synthetic materials.

One example of surgical ligament reconstruction is the surgical treatment of a torn anterior cruciate ligament (ACL), which is the major stabilizing ligament of the knee. An ACL reconstruction typically removes the damaged ACL and replaces it with a graft. The ACL procedure may be performed arthroscopically and generally involves preparing a bone tunnel through the tibia and femur, placing a ligament graft extending between the two bone tunnels and securing each end of the graft within its respective tunnel. One method of ACL reconstruction uses bone-tendon-bone (BTB) ligament grafts, which are harvested from the patella and tibia. Another method employs the use of soft tissue grafts (semitendinosus, hamstring, achilles, quadriceps, etc.). Soft tissue grafts are generally viewed as beneficial in ligament reconstructions. For example, soft tissue grafts do not leave the patella in a weakened state like BTB grafts.

During preparation for ligament reconstruction surgery, the graft material may be trimmed, sized and sutures attached to it to help provide secure fixation of the graft. Suture attachment to the graft is generally completed by the hand sewing of sutures by a variety of methods including baseball whip-stitch, Krakow suture technique, and running stitch. This process may take a surgeon or surgical assistant several minutes for each end of the graft.

There is a need for an efficient method of suture attachment to grafts to reduce the preparation time for ligament reconstruction surgery. There is a need to provide the surgeon or surgical assistant with a reproducible method of suture attachment to assure consistent results. A need thus exists for improved devices and methods for soft tissue graft preparation.

### SUMMARY

The present disclosure relates to a soft tissue graft preparation device including a handle portion, a body portion extending distally from the handle portion, and an end effector operatively connected to a distal end of the body portion. The end effector includes first and second members. The presently disclosed soft tissue graft preparation device is provided with a filamentous material attachment structure that includes at least one filamentous material attached thereto, wherein the filamentous material attachment structure is placed in longitudinal juxtaposition to a tissue contacting surface of the first member and/or a tissue contacting surface of the second member.

The present disclosure also relates to a method of filamentous material attachment to a graft material using an end effector of a surgical device that includes the initial steps of providing the graft material and providing a filamentous material attachment structure that includes at least one filamentous material attached thereto. The method also includes the steps of: positioning the filamentous material attachment structure adjacent a tissue contacting surface of a first member of the end effector; positioning the graft material between a second member of the end effector and the filamentous material attachment structure; moving the first member relative to the second member, thereby bringing the filamentous material attachment structure adjacent the graft material; and implanting mechanical surgical fasteners into the filamentous material attachment structure and the graft using the end effector, thereby affixing the filamentous material attachment structure to the graft.

The present disclosure also relates to another method of filamentous material attachment to a graft material using an end effector of a surgical device. The method includes the initial steps of fabricating a filamentous material attachment structure that includes at least one filamentous material attached thereto and providing a graft material. The method also includes the steps of: positioning the filamentous material attachment structure adjacent a tissue contacting surface of a first member of the end effector; providing a biocompatible film; positioning the biocompatible film adjacent a tissue contacting surface of a second member of the end effector; positioning the graft material between the biocompatible film and the filamentous material attachment structure; moving the first member relative to the second member; and implanting mechanical surgical fasteners into the filamentous material attachment structure, the biocompatible film, and the graft material using the end effector, thereby affixing the filamentous material attachment structure and the biocompatible film to the graft material.

### BRIEF DESCRIPTION OF THE DRAWINGS

Objects and features of the presently disclosed soft tissue graft preparation devices and methods will become apparent to those of ordinary skill in the art when descriptions of various embodiments thereof are read with reference to the accompanying drawings, of which:

FIG. 1 is a perspective view of a surgical stapling device, according to an embodiment of the present disclosure;

FIG. 2 is a side view of a surgical stapling device, according to an other embodiment of the present disclosure;

FIG. 3A is a perspective view of a surgical stapling device, according to yet another embodiment of the present disclosure;

FIG. 3B is an enlarged perspective view of the indicated area of detail A shown in FIG. 3A, illustrating a portion of filamentous material, according to an embodiment of the present disclosure;

FIG. 4 is an enlarged top view of a filamentous material attachment structure, according to an embodiment of the present disclosure;

FIG. 5A is an enlarged cross-sectional view of the indicated area of detail I shown in FIG. 4, illustrating the filamentous material in a first position, according to an embodiment of the present disclosure;

FIG. 5B is an enlarged cross-sectional view of the indicated area of detail I shown in FIG. 4, illustrating the filamentous material disposed in a second position, according to another embodiment of the present disclosure;

FIG. 5C is an enlarged cross-sectional view of the indicated area of detail I shown in FIG. 4, illustrating the filamentous material disposed in a third position, according to yet another embodiment of the present disclosure;

FIG. 6A is an enlarged top view of another embodiment of a filamentous material attachment structure, according to the present disclosure;

FIG. 6B is an enlarged side view of the filamentous material attachment structure of FIG. 6A;

FIG. 7 is an enlarged side view of yet another embodiment of a filamentous material attachment structure, according to the present disclosure;

FIG. 8 is a perspective view of the surgical stapling device of FIG. 1 shown with a filamentous material attachment structure and a film aligned for placement onto the end effector, according to an embodiment of the present disclosure;

FIG. 9A is an enlarged perspective view of the end effector of FIG. 8 shown with the filamentous material attachment structure positioned adjacent the tissue contacting surface of the staple cartridge assembly and the film positioned adjacent the tissue contacting surface of the anvil assembly, according to an embodiment of the present disclosure;

FIG. 9B is an enlarged perspective view of the end effector of FIG. 9A shown with a graft material positioned between the filamentous material attachment structure and the film of FIG. 9A, immediately prior to the firing of the surgical stapling device, according to an embodiment of the present disclosure;

FIG. 10 is enlarged perspective view of the end effector of FIG. 8 shown with a filamentous material attachment structure positioned adjacent the staple cartridge assembly, according to an embodiment of the present disclosure;

FIG. 11 is an enlarged perspective view of the end effector of FIG. 10 shown with a graft material positioned between the filamentous material attachment structure and the anvil assembly, according to an embodiment of the present disclosure;

FIG. 12 is an enlarged perspective view of the end effector of FIG. 11, immediately prior to the firing of the surgical stapling device, according to an embodiment of the present disclosure;

FIG. 13 is a cross-sectional view of a graft material with a filamentous material attachment structure and a film attached, according to an embodiment of the present disclosure;

FIG. 14 is a perspective view of the surgical stapling device of FIG. 1 shown with a filamentous material and a film aligned for placement onto the end effector, according to an embodiment of the present disclosure;

FIG. 15A is an enlarged perspective view of the end effector of FIG. 14 shown with the filamentous material positioned adjacent the tissue contacting surface of the staple cartridge assembly and the film positioned adjacent the tissue contacting surface of the anvil assembly, according to an embodiment of the present disclosure;

FIG. 15B is an enlarged perspective view of the end effector of FIG. 15A shown with a graft material positioned between the filamentous material and the film of FIG. 15A, immediately prior to the firing of the surgical stapling device, according to an embodiment of the present disclosure;

FIG. 16 is a perspective view of the surgical stapling device of FIG. 2 shown with a filamentous material attachment structure a nd a film positioned on the end effector, according to an embodiment of the present disclosure;

FIG. 17 is an enlarged side view of the end effector of FIG. 16, shown with the filamentous material attachment structure positioned adjacent the tissue contacting surface of the staple cartridge assembly and the film positioned adjacent the tissue contacting surface of the anvil assembly, according to an embodiment of the present disclosure;

FIG. 18 is an enlarged side view of the end effector of FIG. 17 shown with a graft material positioned between the filamentous material attachment structure and the film, according to an embodiment of the present disclosure;

FIG. 19 is an enlarged side view of the end effector of FIG. 16 shown with a filamentous material attachment structure positioned adjacent the tissue contacting surface of the staple cartridge assembly and a graft material positioned between the filamentous material attachment structure and the anvil assembly, according to an embodiment of the present disclosure;

FIG. 20 is an enlarged side view of the end effector of FIG. 19, immediately prior to the firing of the surgical stapling device, according to another embodiment of the present disclosure;

FIG. 21 is a cross-sectional view of a graft with filamentous material attachment structure attached, according to an embodiment of the present disclosure;

FIG. 22 is an enlarged side view of another embodiment of an end effector, shown with a filamentous material attachment structure positioned adjacent the tissue contacting surface of a staple cartridge assembly and a graft positioned between the filamentous material attachment structure and an anvil assembly, according to the present disclosure;

FIG. 23 is an enlarged side view of the end effector of FIG. 22, immediately prior to the firing of the surgical stapling device, according to another embodiment of the present disclosure; and

FIG. 24 is a flowchart illustrating a method of filamentous material attachment to a graft using an end effector of a surgical device, according to an embodiment of the present disclosure.

### DETAILED DESCRIPTION

Hereinafter, embodiments of the presently disclosed soft tissue graft preparation devices and methods of filamentous material attachment to soft tissue grafts will be described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the device, or component thereof, closer to the user and the term "distal" refers to that portion of the device, or component thereof, farther from the user.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

As used herein, the term "filamentous material" generally refers to a material used surgically to join tissues. Filamentous materials may be constructed from a variety of materials including, but not limited to, surgical gut; silk; polyolefins such as polyethylene and polypropylene; polyamides; polyesters (including aliphatic polyesters); or any combinations of the above materials adapted for use to join tissues. Filamentous material may be constructed in a monofilament form and as braided structures. Filamentous materials may also be in the form of a core sheath construct. Filamentous material may be knitted or woven with other materials, either absorbable or non-absorbable, to form textiles. Filamentous material also can be made into non-woven materials to form fabrics, such as meshes and felts. Filamentous material may be constructed in tape or ribbon-like structures. Filamentous material may be conditioned, treated and/or processed to make it suitable for use at internal body sites, according to means within the purview of those skilled in the art.

Various embodiments of the present disclosure provide soft tissue graft preparation devices for treating tissue and methods of filamentous material attachment to soft tissue grafts. Embodiments of the presently disclosed soft tissue graft preparation devices are generally suitable for use in connection with ligament reconstruction techniques, for example, anterior cruciate ligament (ACL) reconstruction techniques using soft tissue grafts. The presently disclosed soft tissue graft preparation devices may allow a surgeon to efficiently attach single or multiple filamentous materials to the ends of soft tissues grafts, e.g., semitendinosus, gracilis, or tibialis tendon grafts. Embodiments of the presently disclosed soft tissue graft preparation devices may be suitable for use in connection with autographs, allografts, xenographs and/or synthetic grafts.

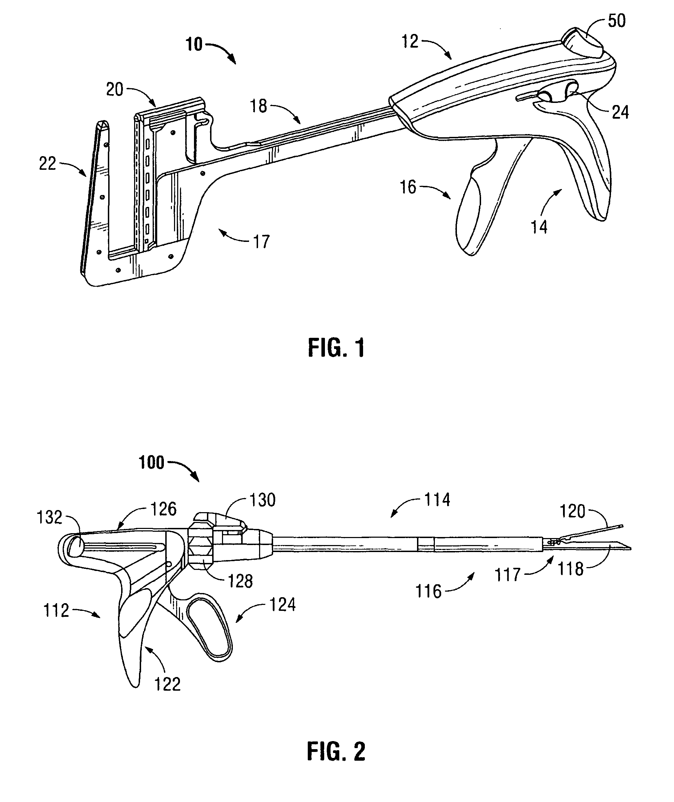

Although the embodiments of filamentous material attachment methods described hereinbelow are targeted toward ACL reconstruction techniques using soft tissue grafts, the presently disclosed methods of filamentous material attachment to soft tissue grafts may be used with other therapies in which grafts are employed, such as, for example, posterior cruciate ligament (PCL) procedures, ligament reconstruction surgeries of the shoulder, elbow and wrist and/or other orthopedic surgeries. Methods of filamentous material attachment to grafts, according to embodiments of the present disclosure, may be used in connection with a variety of surgical stapling devices for implanting mechanical surgical fasteners into tissue. Examples of three different surgical stapling devices for applying an array of surgical staples to tissue are illustrated in FIGS. 1 through 3B. Alternatively, other suitable surgical stapling devices (not shown) may be used.

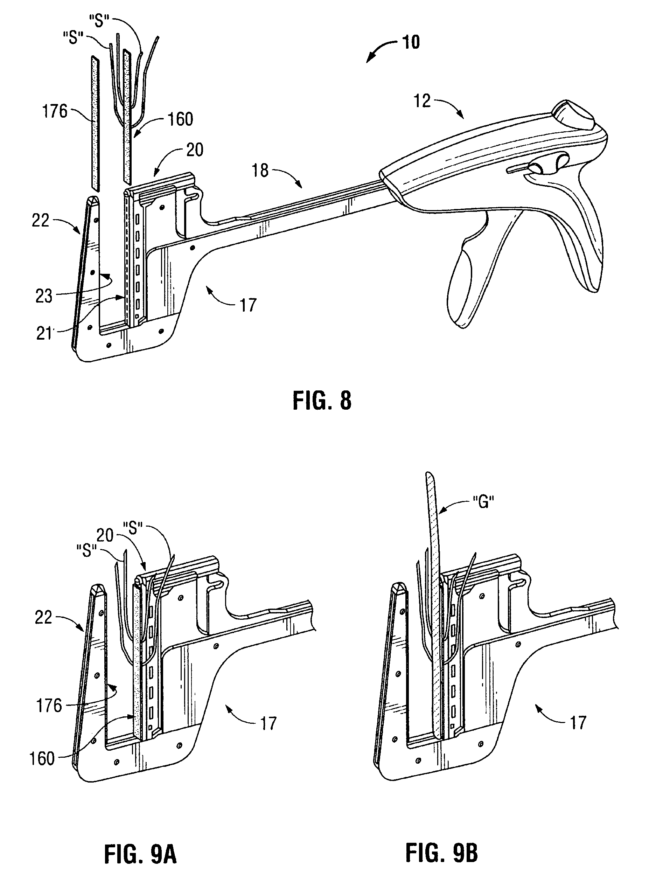

The surgical stapling device shown generally as 10 in FIGS. 1 and 8 includes a body 12 defining a stationary handle 14, a pivotable trigger 16, an elongated central body portion 18, and an end effector 17. The end effector 17 includes a staple cartridge assembly 20 and an anvil assembly 22. It is to be understood that the body portion 18 and the end effector 17 may be configured in a variety of shapes and sizes depending on a particular surgical purpose or to accommodate a particular surgical need. A manual engagement member or thumb button 24 is slidably positioned on each side of the body 12. Thumb buttons 24 are movable to manually advance an alignment pin assembly (not shown). A release button 50 of a release mechanism is positioned on the proximal end of the body 12 and is depressible to allow the staple cartridge assembly 20 to return from an approximated position, wherein the staple cartridge assembly 20 is disposed adjacent to the anvil assembly 22, to a position spaced from the anvil assembly 22 (e.g., as shown in FIGS. 1 and 8). The assemblies of the surgical device 10 and various configurations for each assembly are described in more detail in commonly assigned U.S. Patent No. 7,275,674, issued October 2, 2007, the disclosure of which is herein incorporated by reference in its entirety.

The components of the surgical device 10 are generally formed from thermoplastics including polycarbonates, and metals including stainless steel and aluminum. The particular material selected to form a particular component may depend upon the strength requirements of the particular component. For example, the anvil may be formed from a surgical grade metal, such as stainless steel, and the stationary handle 18 may be formed from a thermoplastic such as polycarbonate. Alternatively, other suitable materials not listed above, which preferably can withstand sterilization procedures, may be used to form components of the stapling device 10, e.g., provided that the materials are suitable for surgical use and meet the strength requirements of the particular component. The surgical stapling device 10 may be used in connection with the presently disclosed methods of filamentous material attachment to soft tissue grafts.

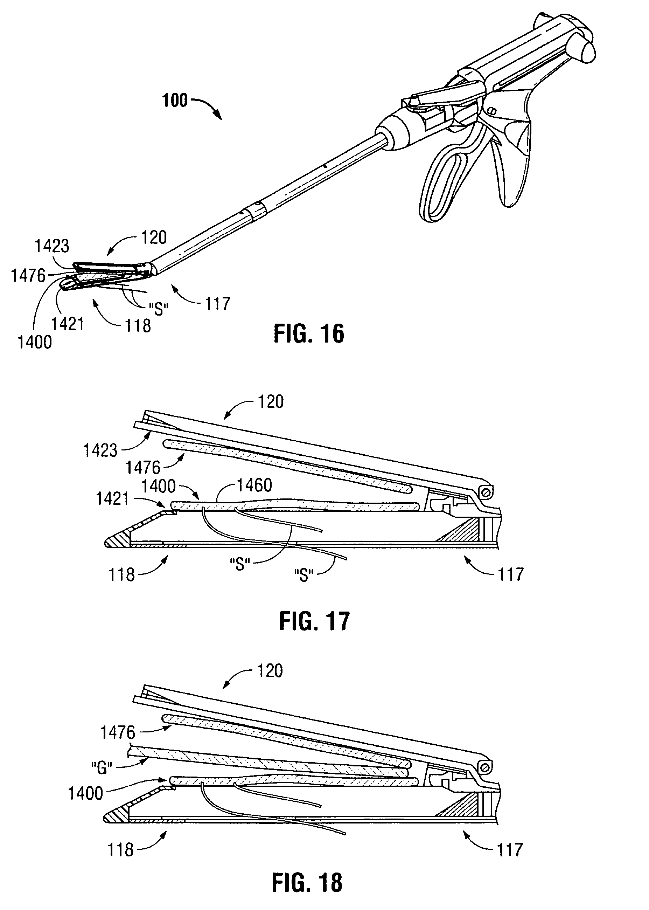

The surgical stapling device shown generally as 100 in FIGS. 2 and 16 includes a handle assembly 112 and an elongated body 114. A disposable loading unit or DLU 116 is releasably secured to a distal end of the elongated body 114. Disposable loading unit 116 includes an end effector 117 having a staple cartridge assembly 118 housing a plurality of surgical staples and an anvil assembly 120 movably secured in relation to the staple cartridge assembly 118. In some embodiments, the disposable loading unit 116 is configured to apply linear rows of staples measuring from about 30 mm to about 60 mm in length. Disposable loading units having linear rows of staples of other lengths are also envisioned, e.g., 45 mm. Handle assembly 112 includes a stationary handle member 122, a movable handle member 124, and a barrel portion 126. A rotatable member 128 may be mounted on the forward end of the barrel portion 126 to facilitate rotation of the elongated body 114 with respect to the handle assembly 112. An articulation lever 130 may also be mounted on the forward end of the barrel portion 126 adjacent to the rotatable knob 128 to facilitate articulation of the end effector 117. A pair of retraction knobs 132 is movably positioned along the barrel portion 126 to return the surgical stapling apparatus 100 to a retracted position. The assemblies of the surgical device 100 and various configurations for each assembly are described in more detail in commonly assigned U.S. Patent Nos. 7,308,998 and 7,303,107, the disclosures of which are herein incorporated by reference in their entireties. The surgical stapling device 100 may be used in connection with the presently disclosed methods of filamentous material attachment to soft tissue grafts.

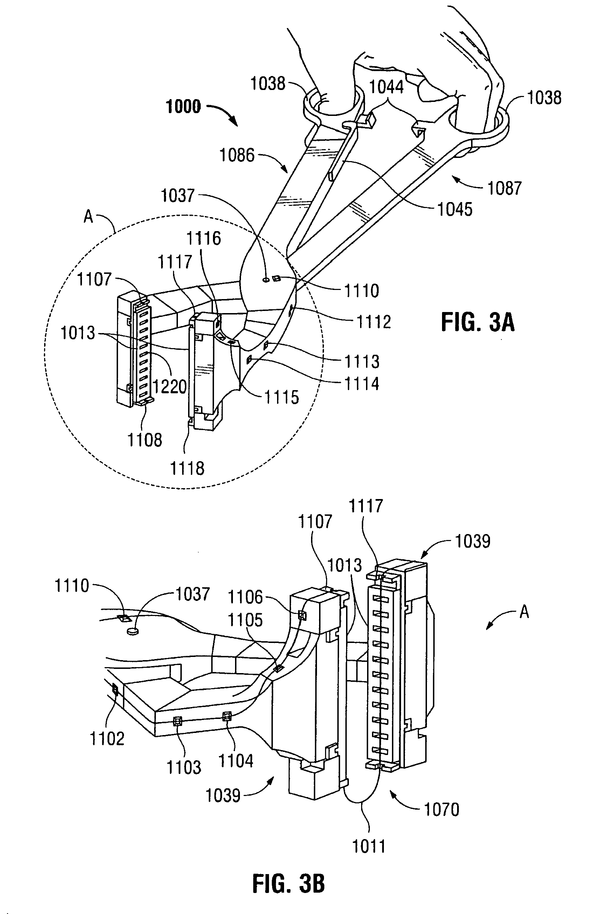

The surgical stapling device shown generally as 1000 in FIGS. 3A and 3B includes handles 1086 and 1087 and a plurality of guides 1102-1106 and 1110-1116 for releasably retaining a portion of filamentous material 1011 substantially adjacent the handles 1086 and 1087. Handles 1086 and 1087 of the stapler 1000 each have proximal and distal ends with stapling assemblies 1013 disposed adjacent the distal ends and finger gripping portions 1038 disposed at the proximal ends thereof. Handles 1086 and 1087 are pivotally connected to each other by pivot pin 1037. Resilient means such as a spring (not shown) may be included in the stapler 1000 to bias handles 1086 and 1087 apart in the absence of a biasing force provided by the user. Stapler 1000 may include a catch mechanism 1044 having a generally known construction disposed on the handles to hold the stapler 1000 in a tissue clamping position after clamping of the handles. In addition, a safety mechanism 1045 is provided at the proximal end of either handles 1086 and 1087 for preventing undesired clamping of the handles. The assemblies of the surgical device 1000 and various configurations for each assembly are described in more detail in commonly assigned U.S. Patent No. 5,490,856, issued February 13, 1996, the disclosure of which is herein incorporated by reference in its entirety. The surgical stapling device 1000 may be used in connection with the presently disclosed methods of filamentous material attachment to soft tissue grafts.

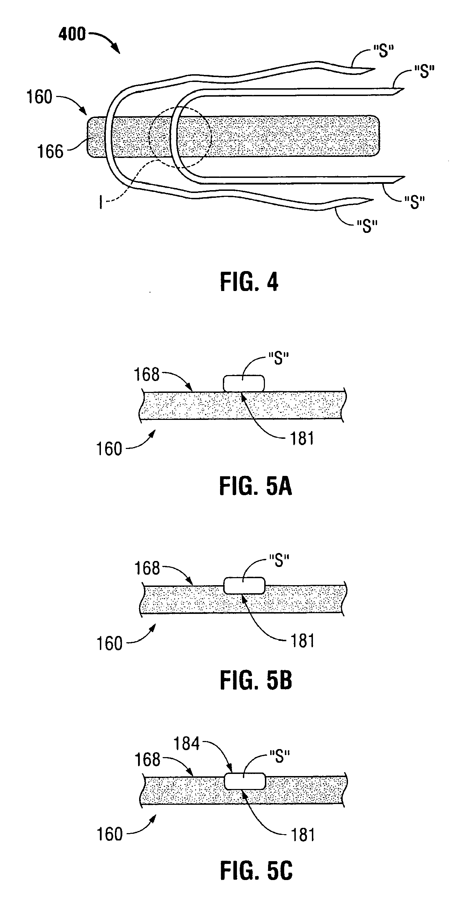

FIG. 4 shows a filamentous material attachment structure, according to an embodiment of the present disclosure. The filamentous material attachment structure 300 includes a thin film 160 with two filamentous materials "S" attached to the film 160. Although two filamentous materials "S" of substantially equal length are shown in FIG. 4, various number and length of filamentous materials may be employed. Each of the filamentous materials "S" bisects the film 160 and outwardly extends from the opposite sides of the film 160. Film 160 may be configured in a variety of forms including a film, ribbon, sheet, tape, or the like. Film 160 may be bioabsorbable, partially bioabsorbable, or non-bioabsorbable. Suitable materials for use in forming the film 160 may include any biocompatible material. Any combination of natural, synthetic, bioabsorbable and/or non-bioabsorbable materials may be used to form the film 160. Film 160 may be formed of a sheet of woven material, non-woven material, mesh material, or a continuous film.

Suitable non-degradable materials include, but are not limited to, polyolefins such as polyethylene (including ultra high molecular weight polyethylene) and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, poly trimethylene terephthalate, and polybutylene terephthalate; polyethers; polytetramethylene ether glycol; polybutesters, including copolymers of butylene terephthalate and polytetramethylene ether glycol; 1,4-butanediol; polyurethanes; acrylic polymers; methacrylics; vinyl halide polymers and copolymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof. Additionally, non-biodegradable polymers and monomers may be combined with each other.

Suitable biodegradable materials that may be utilized to form the film 160 and filamentous materials "S" of the present disclosure include, but are not limited to, aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

More specifically, for the purpose of this invention, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl-1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof. In certain embodiments, the filamentous materials may comprise an aliphatic polyester.

Other suitable biodegradable polymers include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Additionally, synthetically modified natural polymers such as cellulose and polysaccharide derivatives, including alkyl celluloses, hydroxyalkyl celluloses, cellulose ethers, cellulose esters, nitrocelluloses, and chitosan may be utilized. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses."

In embodiments, suitable materials which may be utilized to form the film 160 include homopolymers, copolymers, and/or blends possessing glycolic acid, lactic acid, glycolide, lactide, dioxanone, trimethylene carbonate, caprolactone, and various combinations of the foregoing. For example, in some embodiments, a copolymer of glycolide and trimethylene carbonate may be utilized. Methods for forming such copolymers are within the purview of those skilled in the art and include, for example, the methods disclosed in U.S. Patent No. 4,300,565, the disclosure of which is herein incorporated by reference in its entirety. Suitable copolymers of glycolide and trimethylene carbonate may possess glycolide in amounts from about 60% to about 75% by weight of the copolymer, in embodiments, from about 65% to about 70% by weight of the copolymer, with the trimethylene carbonate being present in amounts from about 25% to about 40% by weight of the copolymer, in embodiments from about 30% to about 35% by weight of the copolymer.

Other suitable materials for forming the film 160 include, in embodiments, copolymers of glycolide, dioxanone and trimethylene carbonate. Such materials may include, for example, copolymers possessing glycolide in amounts from about 55% to about 65% by weight of the copolymer, in embodiments from about 58% to about 62% by weight of the copolymer, in some embodiments about 60% by weight of the copolymer; dioxanone in amounts from about 10% to about 18 % by weight of the copolymer, in embodiments from about 12% to about 16 % by weight of the copolymer, in some embodiments about 14% by weight of the copolymer; and trimethylene carbonate in amounts from about 17% to about 35% by weight of the copolymer, in embodiments from about 22% to about 30% by weight of the copolymer, in embodiments about 26% by weight of the copolymer.

In other embodiments, a copolymer of glycolide, lactide, trimethylene carbonate and ε-caprolactone may be utilized to form the film 160. Such materials may include, for example, a random copolymer possessing caprolactone in amounts from about 14% to about 20% by weight of the copolymer, in embodiments from about 16% to about 18% by weight of the copolymer, in some embodiments about 17% by weight of the copolymer; lactide in amounts from about 4% to about 10% by weight of the copolymer, in embodiments from about 6% to about 8% by weight of the copolymer, in some embodiments about 7% by weight of the copolymer; trimethylene carbonate in amounts from about 4% to about 10% by weight of the copolymer, in embodiments from about 6% to about 8% by weight of the copolymer, in embodiments about 7% by weight of the copolymer; and glycolide in amounts from about 60% to about 78% by weight of the copolymer, in embodiments from about 66% to about 72% by weight of the copolymer, in embodiments about 69% by weight of the copolymer.

In one embodiment, the film 160 is fabricated from "BIOSYN", which is a non-absorbent, biocompatible and bioabsorbable material. "BIOSYN" is made from GLYCOMER 631 (a block copolymer), a synthetic polyester composed of glycolide, dioxanone and trimethylene carbonate. One block of the resulting copolymer contains randomly combined units derived from p-dioxanone (1,4-dioxan-2-one) and trimethylene carbonate (1,3-dioxan-2-one). The second block of the copolymer contains randomly combined units derived from glycolide and p-dioxanone. The resulting polyester is an ABA triblock terpolymer possessing about 60% glycolide, about 14% dioxanone, and about 26% trimethylene carbonate.

In the embodiment depicted in FIG. 4, the film 160 includes a bioabsorbable material 166. Bioabsorbable material 166 may include synthetic bioabsorbable polymers, such as those mentioned above.

Additionally, the film may comprise any or all of emulsifying agents, solubilizing agents, wetting agents, taste modifying agents, plasticizers, active agents, water soluble inert fillers, preservatives, buffering agents, coloring agents, and stabilizers. Addition of a plasticizer to the formulation can improve flexibility. The plasticizer or mixture of plasticizers may be polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums.

The film 160 may include therapeutic agents. Therapeutic agents include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor ), vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; cells, viruses, and ribozymes.

In embodiments, the therapeutic agent may include at least one of the following drugs, including combinations and alternative forms of the drugs such as alternative salt forms, free acid form, free base forms, pro-drugs and hydrates: analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloide, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate); antiasthmatics (e.g., ketotifen and traxanox); antibiotics (e.g., neomycin, streptomycin, chloramphenicol, cephalosporin, ampicillin, penicillin, tetracycline, and ciprofloxacin); antidepressants (e.g., nefopam, oxypertine, doxepin, amoxapine, trazodone, amitriptyline, maprotiline, phenelzine, desipramine, nortriptyline, tranylcypromine, fluoxetine, doxepin, imipramine, imipramine pamoate, isocarboxazid, trimipramine, and protriptyline); antidiabetics (e.g., biguanides and sulfonylurea derivatives); antifungal agents (e.g., griseofulvin, ketoconazole, itraconizole, amphotericin B, nystatin, and candicidin); antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine); anti-inflammatories (e.g., (non-steroidal) indomethacin, ketoprofen, flurbiprofen, naproxen, ibuprofen, ramifenazone, piroxicam, (steroidal) cortisone, dexamethasone, fluazacort, celecoxib, rofecoxib, hydrocortisone, prednisolone, and prednisone); antineoplastics (e.g., cyclophosphamide, actinomycin, bleomycin, dactinomycin, daunorubicin, doxorubicin, epirubicin, mitomycin, methotrexate, fluorouracil, gemcitabine, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, goserelin, leuprolide, tamoxifen, interferon alfa, retinoic acid (ATRA), nitrogen mustard alkylating agents, and piposulfan); antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene); immunosuppressive agents (e.g., cyclosporine, azathioprine, mizoribine, and FK506 (tacrolimus)); antimigraine agents (e.g., ergotamine, propanolol, isometheptene mucate, and dichloralphenazone); sedatives/hypnotics (e.g., barbiturates such as pentobarbital, pentobarbital, and secobarbital; and benzodiazapines such as flurazepam hydrochloride, triazolam, and midazolam); antianginal agents (e.g., beta-adrenergic blockers; calcium channel blockers such as nifedipine, and diltiazem; and nitrates such as nitroglycerin, isosorbide dinitrate, pentearythritol tetranitrate, and erythrityl tetranitrate); antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine); antimanic agents (e.g., lithium carbonate); antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine); antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium); antigout agents (e.g., colchicine, and allopurinol); anticoagulants (e.g., heparin, heparin sodium, and warfarin sodium); thrombolytic agents (e.g., urokinase, streptokinase, and alteplase); antifibrinolytic agents (e.g., aminocaproic acid); hemorheologic agents (e.g., pentoxifylline); antiplatelet agents (e.g., aspirin); anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, mephenytoin, phensuximide, paramethadione, ethotoin, phenacemide, secobarbitol sodium, clorazepate dipotassium, and trimethadione); antiparkinson agents (e.g., ethosuximide); antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorphenirarnine maleate, methdilazine, and); agents useful for calcium regulation (e.g., calcitonin, and parathyroid hormone); antibacterial agents (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antiviral agents (e.g., interferon alpha, beta or gamma, zidovudine, amantadine hydrochloride, ribavirin, and acyclovir); antimicrobials (e.g., cephalosporins such as cefazolin sodium, cephradine, cefaclor, cephapirin sodium, ceftizoxime sodium, cefoperazone sodium, cefotetan disodium, cefuroxime e azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium; penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium; erythromycins such as erythromycin ethylsuccinate, erythromycin, erythromycin estolate, erythromycin lactobionate, erythromycin stearate, and erythromycin ethylsuccinate; and tetracyclines such as tetracycline hydrochloride, doxycycline hyclate, and minocycline hydrochloride, azithromycin, clarithromycin); antiinfectives (e.g., GM-CSF); bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate; anticholinergic agents such as ipratropium bromide; xanthines such as aminophylline, dyphylline, metaproterenol sulfate, and aminophylline; mast cell stabilizers such as cromolyn sodium; inhalant corticosteroids such as beclomethasone dipropionate (BDP), and beclomethasone dipropionate monohydrate; salbutamol; ipratropium bromide; budesonide; ketotifen; salmeterol; xinafoate; terbutaline sulfate; triamcinolone; theophylline; nedocromil sodium; metaproterenol sulfate; albuterol; flunisolide; fluticasone proprionate; steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; and thyroid hormones such as levothyroxine sodium); hypoglycemic agents (e.g., human insulin, purified beef insulin, purified pork insulin, glyburide, chlorpropamide, glipizide, tolbutarnide, and tolazamide); hypolipidemic agents (e.g., clofibrate, dextrothyroxine sodium, probucol, pravastitin, atorvastatin, lovastatin, and niacin); proteins (e.g., DNase, alginase, superoxide dismutase, and lipase); nucleic acids (e.g., sense or anti-sense nucleic acids encoding any therapeutically useful protein, including any of the proteins described herein); agents useful for erythropoiesis stimulation (e.g., erythropoietin); antiulcer/antireflux agents (e.g., famotidine, cimetidine, and ranitidine hydrochloride); antinauseants/antiemetics (e.g., meclizine hydrochloride, nabilone, prochlorperazine, dimenhydrinate, promethazine hydrochloride, thiethylperazine, and scopolamine); as well as other drugs useful in the compositions and methods described herein include mitotane, halonitrosoureas, anthrocyclines, ellipticine, ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. In some embodiments, the drug may be water soluble. In some embodiments, the drug may not be water soluble.

In other embodiments, the film 160 is biological tissue harvested from the patient (autograft) or donors (allograft) or from a species other than man (xenograft).

As described above, filamentous materials "S" may be constructed in a monofilament form and/or braided structures. Filamentous materials "S" are preferably substantially non-toxic, capable of being readily sterilized, have good tensile strength and have acceptable knot-tying and knot-holding characteristics. The shape, size and pattern of the filamentous materials "S" may be varied from the configuration depicted in FIG. 4.

FIGS. 5A through 5C are enlarged side views of the indicated area of detail I shown in FIG. 4, illustrating first, second and third positions of the filamentous materials "S," respectively. The position of the filamentous materials "S" relative to the upper surface 168 of the film 160 may depend on various factors, including but not limited to the density of the film 160, thickness of the film 160, material composition of the film 160, thickness of biocompatible adhesive material (if any), and/or the material composition of the filamentous materials "S."

FIG. 5A shows the filamentous material "S" located in a first position relative to the upper surface 168 of the film 160, wherein the bottom surface 181 of the filamentous material "S" is disposed adjacent to the upper surface 168 of the film 160. Filamentous material "S" may be attached to the film 160, e.g., using a biocompatible adhesive material (not shown), such as an isocyanate, cyanoacrylate, urethane, or other suitable adhesive material. Filamentous material "S" may be woven into the film 160.

FIG. 5B shows the filamentous material "S" located in a second position relative to the upper surface 168 of the film 160, wherein the bottom surface 181 of the filamentous material "S" is disposed below the upper surface 168 of the film 160. The film 160 may include a channel or groove, which is suitably dimensioned to receive a portion of the filamentous material "S" below the upper surface 168 of the film 160. A biocompatible adhesive material (not shown) may be disposed in the channel.

FIG. 5C shows the filamentous material "S" located in a third position relative to the upper surface 168 of the film 160, wherein the bottom surface 181 of the filamentous material "S" is disposed below the upper surface 168 of the film 160, and the upper surface 184 of the filamentous material "S" is disposed substantially flush with the upper surface 168 of the film 160. The position of the filamentous materials "S" relative to the upper surface 168 of the film 160 may be varied from the first, second and third positions depicted in FIGS. 5A through 5C.

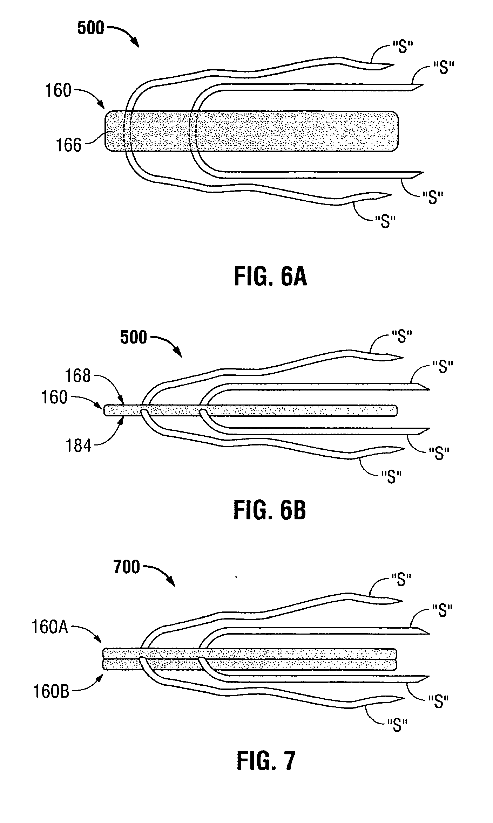

FIGS. 6A and 6B show another embodiment of a filamentous material attachment structure according to the present disclosure. Filamentous material attachment structure 500 of FIGS. 6A and 6B is similar to the filamentous material attachment structure 300 shown in FIG. 4, except the portion of each of the filamentous materials "S" that traverses and bisects the film 160 is contained entirely between the upper and lower surfaces 168, 184 of the film 160. FIG. 6B is a lateral view of the filamentous material attachment structure 500 of FIG. 6A showing the filamentous materials "S" positioned between the upper and lower surfaces 168, 184. In one embodiment of the present disclosure, the filamentous materials "S" are embedded into the film 160 during the fabrication of the film 160. For example, the film 160 may include a base resin and a curing agent, and the filamentous materials "S" may be positioned within the film 160 (e.g., as shown in FIGS. 6A and 6B) prior to a curing step of the fabrication process. Filamentous materials "S" may alternatively, or additionally, be inserted into the film 160 after fabrication of the film 160, e.g., using a piercing instrument such as a needle.

FIG. 7 shows another embodiment of a filamentous material attachment structure according to the present disclosure. Filamentous material attachment structure 700 includes a first film 160A, a second film 160B, wherein the film 160A covers the second film 160B, and two filamentous materials "S", which are attached at the interface between the first and second films 160A, 160B. Either or both of the first and second films 160A, 160B may include bioabsorbable materials. First and second films 160A, 160B may be formed of the same material.

Referring to FIG. 8, the surgical stapling device of FIG. 1 is shown with a filamentous material attachment structure 160 (e.g., shown in FIG.6A) aligned for placement onto the tissue contacting surface 21 of the staple cartridge assembly 20, and a film 176 aligned for placement onto the tissue contacting surface 23 of the anvil assembly 22. Film 176 may include bioabsorbable material. Film 176 may include biocompatible material.

Referring to FIGS. 9A and 9B, the end effector 17 of the surgical stapling device of FIG. 8 is shown with the filamentous material attachment structure 160 positioned adjacent to the tissue contacting surface 21 of the staple cartridge assembly 20, and the film 176 positioned adjacent to the tissue contacting surface 23 of the anvil assembly 22. In FIG. 9B, the end effector 17 is shown with a graft material "G" positioned between the filamentous material attachment structure 160 and the film 176.

Graft material "G" may be rigid, semi-rigid, compliant, resilient, elastic, compressible, expandable, and/or flexible. In some embodiments of the present disclosure, graft material "G" is biological tissue harvested from the patient (autograft) or donors (allograft) or from a species other than man (xenograft). In the embodiment depicted in FIG. 9B, the graft material "G" is formed from a biocompatible material of natural origin, and may be bioabsorbable or non-bioabsorbable. Examples of suitable materials for forming the graft material "G" include autograft, allograft or xenograft; tissue materials including soft tissues, connective tissues, demineralized bone matrix and combinations thereof. Other examples of suitable materials for forming the graft material "G" may include bioabsorbable materials or non-bioabsorbable materials including those listed above.

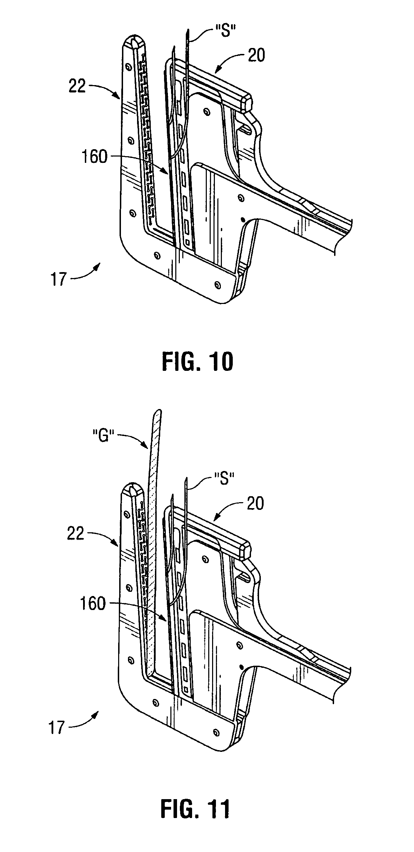

In some embodiments of the present disclosure, the film 176 is omitted. For example, as cooperatively shown in FIGS. 10 and 11, the end effector 17 of the surgical stapling device of FIG. 8 may be provided with a filamentous material attachment structure 160 positioned adjacent to the staple cartridge assembly 20, and a graft material "G" may be positioned between the filamentous material attachment structure 160 and the anvil assembly 22.

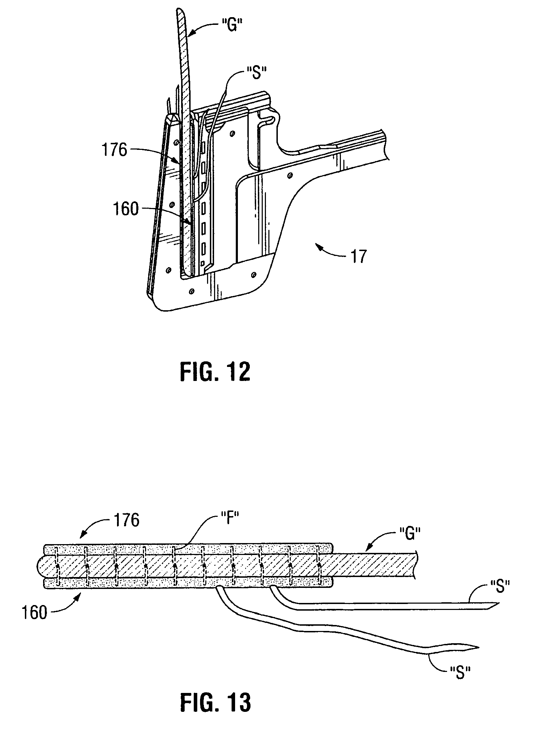

FIG. 12 shows the end effector 17 of FIGS. 9A and 9B, immediately prior to the firing of the surgical stapling device 10. FIG. 13 shows the graft material "G" with the filamentous material attachment structure 160 and the film 176 attached by a plurality of mechanical surgical fasteners "F", e.g., using the surgical stapler device of FIGS. 1 and 8.

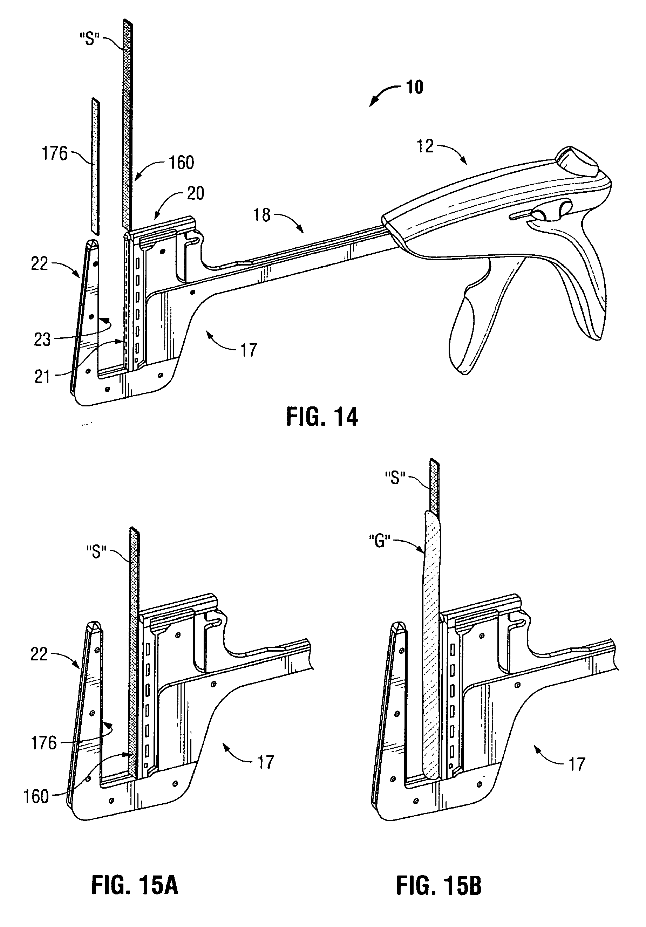

Referring to FIG. 14, the surgical stapling device 10 of FIG. 1 is shown and includes a filamentous material "S" and a film 176 aligned for placement onto the end effector. FIG. 15A is an enlarged view of the end effector 17 of FIG. 14 shown with the filamentous material "S" positioned adjacent the tissue contacting surface 21 of the staple cartridge assembly 20 and the film 176 positioned adjacent the tissue contacting surface 23 of the anvil assembly 22. Film 176 may be omitted. FIG. 15B is a view of the end effector of FIG. 15A shown with a graft material "G" positioned between the filamentous material "S" and the film 176 of FIG. 15A, immediately prior to the firing of the surgical stapling device 10.

Referring to FIG. 16, the surgical stapling device of FIG. 2 is shown with a filamentous material attachment structure 1400 positioned substantially adjacent to the tissue contacting surface 1421 of the staple cartridge assembly 118, and a film 1476 positioned substantially adjacent to the tissue contacting surface 1423 of the anvil assembly 120. FIGS. 17 and 18 are enlarged views of the end effector 117 of FIG. 16. As shown in FIG. 17, the filamentous material attachment structure 1400 includes a film 1460 and two filamentous materials "S." Filamentous material attachment structure 1400 is similar to filamentous material attachment structure 160 shown in FIG. 6A, and further description of the filamentous material attachment structure 1400 is omitted in the interests of brevity.

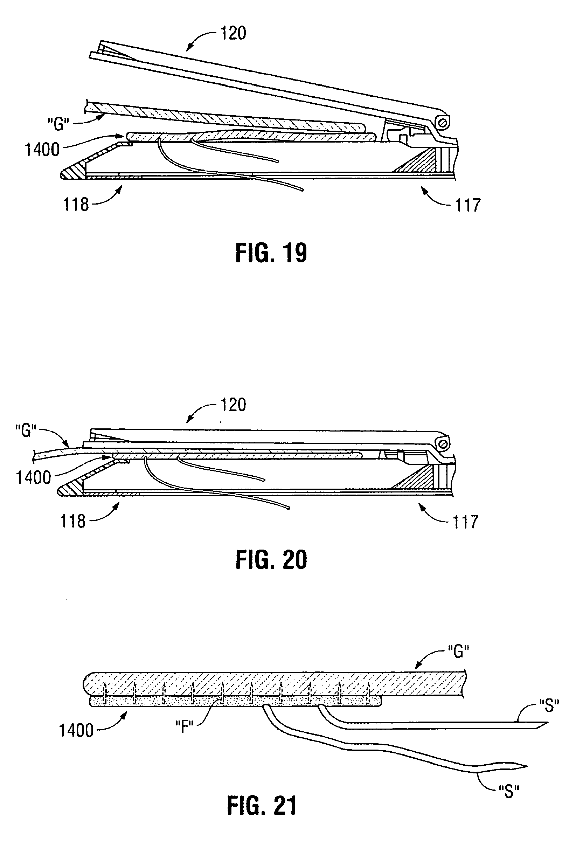

Referring to FIG. 18, the end effector 117 is shown with a graft material "G" positioned between the filamentous material attachment structure 1400 and the film 1476. Film 1476 may be omitted (see, e.g., FIGS. 19 and 20).

FIGS. 19 and 20 are enlarged views of the end effector 117 of the surgical stapler device of FIG. 16. In FIG. 19, the end effector 117 is shown with the filamentous material attachment structure 1400 positioned substantially adjacent to the tissue contacting surface of the staple cartridge assembly 118, and a graft material "G" positioned between the filamentous material attachment structure 1400 and the anvil assembly 120. FIG. 20 illustrates the end effector 117 shown in FIG. 19 immediately prior to the firing of the surgical stapling device.

FIG. 21 shows the graft material "G" with the filamentous material attachment structure 1400 attached by a plurality of mechanical surgical fasteners "F", e.g., using the surgical stapler device of FIGS. 2 and 16.

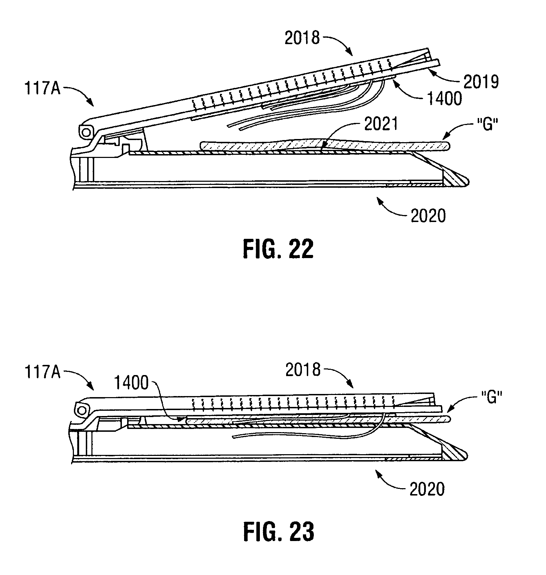

FIGS. 22 and 23 show an end effector 117A provided with a filamentous material attachment structure 1400 and a graft material "G". The end effector 117A of FIGS. 22 and 23 is similar to the end effector 117 shown in FIGS. 17 through 20, except that the relative positions of the anvil assemblies and the stable cartridge assemblies are reversed. In FIGS.17 through 20, the anvil assembly 120 is the upper member of the end effector 117 and the stable cartridge assembly 118 is the lower member of the end effector 117. In the alternative embodiment shown in FIGS. 22 and 23, the anvil assembly 2020 is the lower member of the end effector 117A and the stable cartridge assembly 2018 is the upper member of the end effector 117A.

Referring to FIG. 23, the end effector 117A of FIG. 22 is shown with the filamentous material attachment structure 1400 positioned substantially adjacent to the tissue contacting surface 2019 of the staple cartridge assembly 2018, and a graft material "G" positioned between the filamentous material attachment structure 1400 and the tissue contacting surface 2021 of the anvil assembly 2020, immediately prior to the firing of the surgical stapling device.

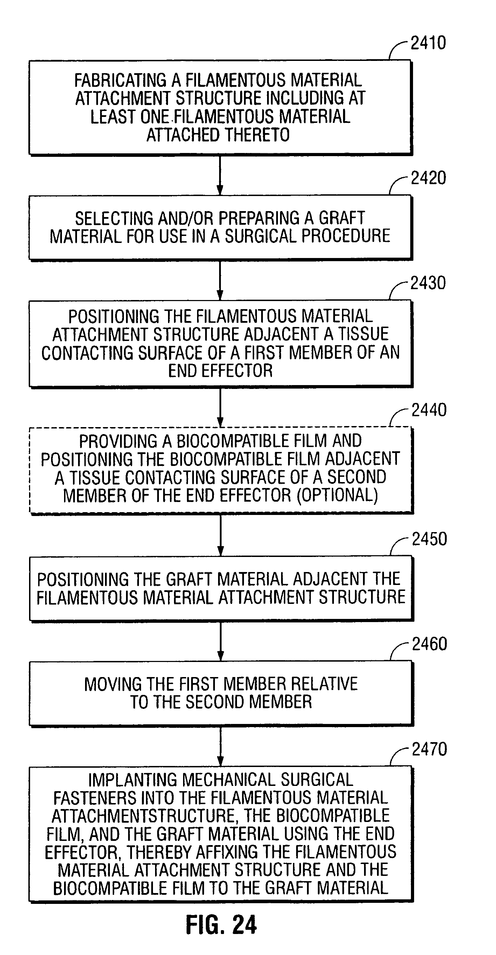

FIG. 24 is a flowchart illustrating a method of filamentous material attachment to a graft material using an end effector (e.g., 17 shown in FIG. 1 and FIGS. 8 through 12) of a surgical device (e.g., 10 shown in FIGS. 1 and 8), according to an embodiment of the present disclosure. It is to be understood that the steps of the method provided herein may be performed in combination and in a different order than presented herein without departing from the scope of the disclosure.

In step 2410, a filamentous material attachment structure (e.g., 500 shown in FIG. 6A) is fabricated including a biocompatible film (e.g., 160 shown in FIG. 6A) with at least one filamentous material (e.g., "S" shown in FIG. 6A) attached to the biocompatible film.

In step 2420, a graft material (e.g., "G" shown in FIG. 9B) is selected and/or prepared for use in a surgical procedure. The graft material may be formed of any biocompatible material of natural origin. The graft material may be a soft tissue graft. The graft material may include an autograft, an allograft or a xenograft. The graft material may include a synthetic material or combinations of natural and synthetic material. An example of a material that may be suitable for use as the graft material is a polyester mesh material commercially available under the trademark PARIETEX^{™} Composite (PCO) offered by Tyco Healthcare Group LP (d/b/a Covidien).

In step 2430, the filamentous material attachment structure is positioned adjacent a tissue contacting surface of a first member of the end effector. The first member of the end effector may be a staple cartridge assembly (e.g., 20 shown in FIGS. 1 and 8).

In optional step 2440, a biocompatible film (e.g., 176 shown in FIG. 8) is provided and positioned adjacent a second member of the end effector. Optional step 2440 may be included depending on a particular surgical purpose or to accommodate a particular surgical need.

In step 2450, the graft material is positioned between the biocompatible film and the filamentous material attachment structure (e.g., 500 shown in FIG. 6A), e.g., adjacent the filamentous material attachment structure. In a case where the optional step 2440 is omitted, the graft material may be positioned adjacent the tissue contacting surface of the second member of the end effector. The second member of the end effector may be an anvil assembly (e.g., 22 shown in FIGS. 1 and 8) operatively associated with the staple cartridge assembly (e.g., 20 shown in FIGS. 1 and 8). The graft material may be aligned with the second member of the end effector to facilitate fastener placement within a center portion of the graft material.

In step 2460, the first member is moved relative to the second member, bringing the filamentous material attachment structure and the biocompatible film adjacent to the graft material. In a case where the optional step 2440 is omitted, moving the first member relative to the second member, in step 2460, brings the filamentous material attachment structure adjacent to the graft material.

In step 2470, a plurality of mechanical surgical fasteners (e.g., "F" shown in FIG. 13) from the staple cartridge assembly are implanted into the filamentous material attachment structure, the biocompatible film, and the graft material using the end effector, thereby affixing the filamentous material attachment structure and the biocompatible film to the graft material. If the optional step 2440 is omitted, a plurality of mechanical surgical fasteners from the staple cartridge assembly are implanted into the filamentous material attachment structure and the graft material using the end effector, in step 2470, thereby affixing the filamentous material attachment structure to the graft material.

Although embodiments of the present disclosure have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. A soft tissue graft preparation device, comprising:
a handle portion;
a body portion extending distally from the handle portion;
an end effector operatively connected to a distal end of the body portion, the end effector including first and second members; and
a filamentous material attachment structure including at least one filamentous material attached thereto, the filamentous material attachment structure placed in longitudinal juxtaposition to at least one of a tissue contacting surface of the first member and a tissue contacting surface of the second member.

2. The soft tissue graft preparation device of claim 1, wherein the second member is operatively associated with the first member, the first and second members being movably connected to one another to bring one into longitudinal juxtaposition relative to the other.

3. The soft tissue graft preparation device of claim 1 or claim 2, wherein the soft tissue graft preparation device is a surgical stapler and the first member is a staple cartridge assembly and the second member is an anvil assembly operatively associated with the staple cartridge assembly.

4. The soft tissue graft preparation device of any preceding claim, wherein the filamentous material attachment structure further includes a film with the at least one filamentous material attached to the film.

5. The soft tissue graft preparation device of claim 4, wherein the film includes bioabsorbable polymers, or the soft tissue graft preparation device of claim 4, wherein the film includes non-bioabsorbable polymers.

6. The soft tissue graft preparation device of claim 4, further comprising a bioabsorbable adhesive for attaching the at least one filamentous material to the film.

7. The soft tissue graft preparation device of claim 4, wherein the film includes at least one channel formed therein, and wherein the at least one filamentous material is at least partially contained within the at least one channel in the film, preferably further comprising an adhesive disposed in the channel.

8. A method of filamentous material attachment to a graft material using an end effector of a surgical device, the method comprising the steps of:
providing a graft material;
providing a filamentous material attachment structure including at least one filamentous material attached thereto;
positioning the filamentous material attachment structure adjacent a tissue contacting surface of a first member of the end effector;
positioning the graft material between a second member of the end effector and the filamentous material attachment structure;
moving the first member relative to the second member, thereby bringing the filamentous material attachment structure adjacent to the graft material; and
implanting mechanical surgical fasteners into the filamentous material attachment structure and the graft material using the end effector, thereby affixing the filamentous material attachment structure to the graft material.

9. The method of claim 8, wherein the filamentous material attachment structure further includes a first film with the at least one filamentous material attached to the first film.

10. The method of claim 8, wherein the first member of the end effector is a staple cartridge assembly and/or the method of claim 8, wherein the second member of the end effector is an anvil assembly operatively associated with the staple cartridge assembly.

11. The method of any of claims 8 to 10, further comprising the steps of:
providing a second film; and
positioning the second film adjacent a tissue contacting surface of a second member of the end effector.

12. The method of any of claims 8 to 11, further comprising the step of:
aligning the graft material to facilitate surgical fastener placement within a center portion of the graft.

13. The method of any of claims 8 to 12, wherein the step of providing the graft material includes at least one of selecting and preparing the graft material for use in a surgical procedure.

14. The method of any of claims 8 to 13, wherein the graft material is a soft tissue graft.

15. The method of any of claims 8 to 14, wherein the graft material includes a biological tissue harvested from at least one of a patient, a donor, and a species other than man.

16. A method of filamentous material attachment to a graft material using an end effector of a surgical device, the method comprising the steps of:
fabricating a filamentous material attachment structure including at least one filamentous material attached thereto;
providing a graft material;
positioning the filamentous material attachment structure adjacent a tissue contacting surface of a first member of the end effector;
providing a biocompatible film;
positioning the biocompatible film adjacent a tissue contacting surface of a second member of the end effector;
positioning the graft material between the biocompatible film and the filamentous material attachment structure;
moving the first member relative to the second member; and
implanting mechanical surgical fasteners into the filamentous material attachment structure, the biocompatible film, and the graft material using the end effector, thereby affixing the filamentous material attachment structure and the biocompatible film to the graft material.
